Europäisches Patentamt

European Patent Office

Office européen des brevets

(11) Publication number: **0 465 556 B1**

(12) **EUROPEAN PATENT SPECIFICATION**

(45) Date of publication of patent specification: **28.06.95**  (51) Int. Cl.⁶: **A61K 47/48**

(21) Application number: **90905802.6**

(22) Date of filing: **03.04.90**

(86) International application number:
**PCT/US90/01773**

(87) International publication number:
**WO 90/11783 (18.10.90 90/24)**

(54) **PLATELET SPECIFIC IMMUNOCONJUGATES.**

(30) Priority: **03.04.89 US 332874**

(43) Date of publication of application:
**15.01.92 Bulletin 92/03**

(45) Publication of the grant of the patent:
**28.06.95 Bulletin 95/26**

(84) Designated Contracting States:
**AT BE CH DE DK ES FR GB IT LI LU NL SE**

(56) References cited:
**EP-A- 187 658**

**Circulation, volume 77, no. 3, 1988, (New York, US), H.K. Gold et al.: "Rapid and sustained coronary artery recanalization with combined bolus injection of recombinant tissue-type plasminogen activator and monoclonal antiplatelet GPIIb/IIIa antibody in a canine preparation", pages 670-677**

**Biochemistry vol. 27, 1988, (Washington, D.C. US), M.S. Runge et Al, pages 1153-1157, abstract page 1153**

(73) Proprietor: **CENTOCOR, INC.**
**244 Great Valley Parkway**
**Malvern, PA 19355 (US)**

(72) Inventor: **BODE, Christoph**
**Albert-Fritz-Strasse 47A**
**D-6900 Heidelberg (DE)**
Inventor: **HABER, Edgar**
**RD2 Pretty Brook Road**
**Princeton, NJ 08540 (US)**
Inventor: **MEINHARDT, Gabriel**
**2466 Hidden Valley Road**
**La Jolla, CA 92037 (US)**

(74) Representative: **Holdcroft, James Gerald, Dr. et al**
**Graham Watt & Co.,**
**Riverhead**
**Sevenoaks, Kent TN13 2BN (GB)**

## Description

Background of the Invention

Platelet aggregation is an essential event in the formation of arterial blood clots (thrombi). Under normal circumstances, blood clots serve to prevent the escape of blood cells and fluid from the vascular system. However, during certain disease states (e.g., myocardial infarction), clots can restrict or totally prevent blood flow, resulting in cellular necrosis.

Coronary arteriographic studies indicate that 87% of transmural myocardial infarctions are caused by coronary thrombosis. DeWood, M.A. et al., N. Eng. J. Med., 303:897-902 (1980). Currently available thrombolytic agents can lyse coronary artery thrombi in the early hours of thrombosis in 50-75% of cases and thereby diminish myocardial injury. However, their clinical application has been attended by significant problems.

Streptokinase and urokinase are plasminogen activators which activate the proenzyme plasminogen, to the fibrinolytic enzyme plasmin which lyses fibrin in clots. However, plasmin is non-selective and not only effects lysis of the fibrin in the thrombus but also promotes generalized fibrinogenolysis, at times resulting in severe bleeding. Laffel, G.L. et al., ibid, 311:71-717 (1984). In addition, the plasmin formed in circulating blood is neutralized rather quickly and is lost for useful thrombolysis. Residual plasmin will degrade several clotting factor proteins, for example, fibrinogen, factor V and factor VIII, thus increasing the potential for hemorrhage . In addition, streptokinase is strongly antigenic and patients with high antibody titers respond inefficiently to treatment and cannot remain on continuous treatment and allergic reactions including shock have been described.

The recent availability of tissue plasminogen activator (tPA) and single-chain urokinase plasminogen activator (scuPA) has somewhat improved the therapeutic prospects, because they are more fibrin-specific. However, at the high dosages needed for effective clot lysis, specificity is partially lost. Therefore bleeding complications similar to those found with streptokinase have been observed.

In addition, a major complication associated with fibrinolysis through the use of currently available plasminogen activators is that after the clot has been lysed, the platelets tend to reaggregate. This results in reocclusion and further heart damage.

It would be useful to improve the efficacy and specificity of thrombolytic therapy by targetting a fibrinolytic agent directly to arterial thrombi in a way that avoids the problems associated with current methods of therapeutic fibrinolysis.

Summary of the Invention

This invention pertains to immunoconjugates comprising a platelet-specific monoclonal antibody or fragments thereof bound to a thrombolytic agent and to their use in antithrombotic therapy. The antibodies can be specific for the GPIIb/IIIa receptor (which is involved in platelet aggregation), or other platelet components. These antibodies bind to platelets and thereby block platelet aggregation. The immunoconjugates of the present invention, which bind to platelets rather than fibrin are selective for arterial clots (e.g., acute myocardial infarcation, stroke) as opposed to venous clots (e.g., pulmonary emboli, deep vein thromboses (DVT)).

Human antibodies or "chimeric antibodies" containing small fractions of a mouse antibody that contain the hypervariable region, are preferred because they minimize some of the immunogenicity problems associated with antibodies composed of nonhuman protein. The thrombolytic moiety of the immunoconjugates of this invention can be urokinase, streptokinase, tissue-type plasminogen activator (tPA), single-chain urokinase plasminogen activator, single-chain streptokinase, acyl-plasminogen-streptokinase activator complex, recombinant variants of these compounds, or fractions containing the enzymatically active portion.

Detailed Description of the Invention

The present invention relates to immunoconjugates comprising platelet-specific monoclonal antibodies or fragments thereof linked to a thrombolytic agent. Platelets make up a large part of arterial thrombi whereas there are only a few platelets in venous thrombi. The immunoconjugates of the present invention direct thrombolytic agents to platelets rather than to fibrin (which is present in both clots, but more is present in venous clots) and thus offers selectivity for arterial clots (e.g., acute myocardial infunction and stroke). The platelet specific immunoconjugates of the present invention are designed for lysis of arterial clots, whereas venous clots (e.g., pulmonary emboli, and DVT) are better treated with fibrin specific agents.

In preferred embodiments, the antibodies are human monoclonal antibodies involved in platelet aggregation, such as the GPIIb/IIIa receptor. The immunoconjugates bind to platelets, thus selectively delivering the thrombolytic agent to the thrombus. The bound immunoconjugates also prevent platelet reaggregation and thrombus reformation.

The immunoconjugates of this invention are specific for platelet surface components. Preferred antibodies for making the immunoconjugates are specific for platelet GP IIb/IIIa receptor. When the antibodies bind to the GPIIb/IIIa receptor, they block ligand binding to the platelet. The antibodies can also be specific for either the GPIIb or GPIIIa components individually. Alternatively, antibodies specific for other platelet antigens can also be employed (e.g., human antibodies that bind to platelet granule membrane protein GMP-140).

In general, platelet-specific antibodies can be prepared by obtaining lymphoid cells from a verterbrate producing antibody against a platelet antigen. The lymphoid cells are fused to immortalizing cells to produce continuous hybrid cell lines.

Preferred lymphoid cells are B lymphocytes which can be obtained from an individual. The lymphoid cells can be obtained from spleen, lymph nodes or peripheral blood by venipuncture or pheresis. The lymphoid cells can be enriched by use of a one step gradient such as Ficoll-Hypaque. The recovered cells can be washed to thoroughly remove the gradient material which may be toxic.

Unwanted or undesirable cell populations such as suppressor cells (CD8$^+$) or B cells making an unwanted isotype such as IgM can be removed. This may be accomplished by complement mediated lysis, cell sorting using flow cytometry or affinity purification such as 'panning'.

Prior to fusion, the B cells can be stimulated with antigen, lymphokines and/or other mitogenic substances or substances that will induce the B cells to synthesize and secrete antibody. Another method of forming the antibody-secreting cells for fusion is by using lymphoid cells from patients with Glanzmann's thrombastheuia, a disease in which the GPIIb/IIIa complex is not expressed. This method is described in copending application, Serial No. 278,805 (H. Lazarus and B.S. Coller, December 1, 1988).

The appropriately stimulated cells are then fused using standard procedures. Kohler and Milstein, Nature, 256: 495-497 (1975); Olsson and Kaplan, Proc. Natl. Acad. Sci. USA, 77: 5429 (1980). The fusion partner is a cell or hybrid of B cell lineage capable of supporting the synthesis and secretion of human antibodies.

Generally, the immortalizing cell line is a tumor cell, which endows the hybridoma with the ability to grow permanently in culture. This ensures a stable culture of antibody-producing hybridoma cells which can produce monoclonal antibodies in a continuous supply. The immortalizing cell may be a plasmacytoma cell, such as a myeloma cell. The myeloma cell can be human, non-human, or a heteromyeloma. Suitable human immortalizing cell lines include the HMMA2.11, HF2, and the U-266 cell lines. A heteromyeloma is a myeloma hybrid formed by the fusion of cells of two different species. See Oestberg, U.S. Patent 4,634,664.

The hybridomas are then screened for production of antibodies reactive with platelets or platelet components such as the GPIIb/IIIa receptor. The screening can be accomplished by an enzyme immunoassay. For example, purified GPIIb/IIIa can be bound to a solid phase. The solid phase can then be contacted with hybridoma supernatant and antibody binding to the GPIIb/IIIa-solid phase can be evaluated with enzyme-conjugated anti-human antibody. Hybridomas that secrete reactive antibodies are cloned.

Another method of forming the antibody-producing cells is by viral or oncogenic transformation. For example, human B-lymphocyte which produced a platlet-specific antibody may be infected and transformed with a virus, such as the Epstein-Barr virus, to give an immortal antibody-producing cell. See, e.g., Kozbor and Roder (1983) Immunology Today, 4(3):72-79. Alternatively, the B-lymphocyte may be transformed by a transforming gene or gene product.

The platelet-specific monoclonal antibody or antibody fragment (e.g., Fab, Fab', F(ab')$_2$, etc.) is conjugated to a thrombolytic agent to form the immunoconjugate of the invention. The term "thrombolytic agent" as used herein is meant to include broadly any agent utilized for or inducing or initiating the lysis of a thrombus. The most common agents are urokinase, streptokinase, tissue-type plasminogen activator (tPA), single chain streptokinase, acyl-plasminogen-streptokinase activator complex, or recombinant variants of these agents.

The term "conjugate" as used herein is meant to include broadly the firm attachment of the thrombolytic agent to the antiplatelet antibody. The attachment may be noncovalent but is preferably covalent. For example, recombinant techniques may be used to covalently attach thrombolytic agent peptides to the hinge region of the antiplatelet antibody heavy chain. This is accomplished by joining the gene coding for the antiplatelet antibody heavy chain with the gene coding for the thrombolytic agent peptide and transfecting this construct into a myeloma cell capable of producing only antibody light chain.

Schnee et al., Proc. Nat. Acad. Sci USA 84: 6904-6908 (1987) and Neuberger et al., Nature 312: 604-608 (1984). The work of Schnee and Neuberger also demonstrates that the activity of the peptide attached to the carboxyl end of the antibody heavy chain in the recombinantly produced conjugates approaches normal levels, even when the peptides activity depends on multiple intrachain disulfide bonds.

The coupling of the two entities may also be accomplished via a coupling or conjugating agent. There are several intermolecular cross-linking reagents which can be utilized (see, for example, Means, G.E. and Feeney, R.E., Chemical Modification of Proteins, Holden-Day, 1974, pp. 39-43). Among these reagents are, for example, N-succinimidyl 3-(2-pyridyldithio) propionate (SPDP) or N, N'-(1,3-phenylene) bismaleimide (both are highly specific for sulhydryls, and form irreversible linkages); N, N'-ethylene-bis-(iodoacetamide) or other such reagent having 6 to 11 carbon methylene bridges (relatively specific for sulfhydryl groups); 1,5-difluoro-2,4-dinitrobenzene (forms irreversible linkages with amino and tyrosine groups); p,p'-difluoro-m, m'-dinitrodiphenylsufone (forms irreversible crosslinkages with amino and phenolic groups); dimethyl adipimidate (specific for amino groups); phenol-2,4-disulfonylchloride (reacts principally with amino groups); hexamethylenediisocyanate or diisothiocyanate, or azophenyl-p-diisocyanate (reacting principally with amino groups); glutaraldehyde (reacting with several different side chains) and disdiazobenzidine (reacting primarily with tyrosine and histidine). These are only a few of several cross-linking agents that can be utilized.

The immunoconjugates can be used as pharmaceutical compositions. For example, the conjugates can be formulated in appropriate compositions by including activating amounts of desired product together with pharmacologically appropriate carriers. Generally speaking, these carriers include aqueous or alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers, such as those based on Ringer's dextrose, and the like. Preservatives and other additives can also be present, such as, for example, antimicrobials, antioxidants, chelating agents, and inert gases. See, generally, Remington's Pharmaceutical Sciences, 16th Ed., Mack, ed. 1980.

The route for administering pharmaceutical compositions prepared according to the method of the invention may be any of those commonly known to those of ordinary skill in the art.

For thrombolytic therapy, the coupled immunoconjugates of the invention can be administered to any patient in need of such therapy. The administration can be by any appropriate mode, including parenteral, intravenous, intramuscular, intraperitoneal or, if appropriate, by direct infusion with a catheter, such as in intracoronary administration. The dosage and frequency of administration will depend on age, sex and condition of the patient, concurrent administration of other drugs, counter indications and other parameters to be taken into account by the clinician. The exact dosage and frequency with which the immunoconjugate of the invention would be administered when used as therapeutic compositions are known to those of ordinary skill in the art or are readily ascertainable with only minor experimentation.

For example, when pharmaceutical compositions of the invention are used in therapy, the dosages and frequency of administration can be comparable to those used for the administration of the thrombolytic agent of the monoclonal antibody itself in the prior art. Generally, however, the dosage will be about 0.05 times the dosage normally utilized for the administration of the thrombolytic agent by itself.

In preferred embodiments of this invention, urokinase (UK) or tissue-type plasminogen activator (tPA) is coupled to the Fab' fragment of a monoclonal antibody (7E3) that selectively binds to platelet membrane glycoprotein GPIIb/IIIa. The desired conjugates (UK-AB and tPA-AB) are formed by disulfide exchange and purified by a two step affinity procedure using Benzamidine Sepharose and anti-mouse-Fab-Sepharose.

The functions of a plasminogen activator and 7E3 are combined in one molecule and thereby target the plasminogen activator to the site of a platelet rich clot. Activator targeting results in markedly enhanced plasmin generation in the vicinity of platelets and enhanced thrombolysis in vitro.

This invention is illustrated further by the following examples.

Example I: Preparation of Immunoconjugates

A. Materials:

Predominantly single chain recombinant tissue-type plasminogen activator (rtPA) (ACTILYSE R) was purchased from Thomae GmbH Biberach, FRG. High molecular weight two-chain urokinase (UROKINASE MEDAC R) was bought from Medac GmbH, Hamburg, FRG. L-Pyroglutamyl-glycyl-L-arginine-p-nitroanilide hydrochloride (S-2444), a chromogenic substrate for urokinase, was obtained from KabiVitrum, as were H-D-isoleucyl-L-prolyl-L-arginine-p-nitroanilide-dihydrochloride (S-2288), a chromogenic substrate for serine proteases, and H-D-Valyl-L-leucyl-L-lysine-p-nitroanilide dihydrochloride, a chromogenic substrate for plasmin (S-2251). Affinity purified polyclonal rabbit anti-human albumin IgG was purchased from Boehringer Mannheim.

N-Succinimidyl-3-(2-pyridyldithio)propionate (SPDP) was purchased from Pierce Chemical (Rockford, IL, USA), 125-I-labeled human fibrinogen from Amersham Buchler GmbH, Braunschweig, FRG. Fresh frozen plasma and platelet rich plasma were purchased from the University of Heidelberg blood bank or from the German Red Cross, Baden-Baden, FRG.

Plasminogen was purified from fresh frozen plasma on lysine-Sepharose® according to established procedures. Deutch DG, Mertz ET, Science, 170:1095-1096 (1970). Purity was assessed by SDS-PAGE and by chromogenic substrate assay (S-2251). Bio-Beads SM2 were obtained from Bio-Rad p-Aminobenzamidine was coupled to CH-Sepharose 4B® as described by Holmberg et al., Biochim. Biophys. Acta, 445:215-222 (1976). Monoclonal antibody 7E3 (Fab')2 was a gift from Centocor, Malvern, PA, USA. All other chemicals came from Sigma (St. Louis, MO, USA).

### B. Purification of Glycoprotein GP IIb/IIIa

GP IIb/IIIa was purified from platelet rich plasma as described. Fitzgerald, L.A. et al., Anal. Biochem., 151:169-177 (1985). Before using the protein in the assay procedures, Triton X-100® was quantitatively removed by gel filtration on a Bio-Beads SM-2 column. Holloway, P.W., Anal. Biochem., 53:304-308 (1973). Alternatively, GP IIb/IIIa was purified on a Lysine-Sepharose 4B column to which 7E3-Fab' had been coupled via SPDP. Purity of the resulting protein preparation was assessed by sodium dodecyl sulphate-polyacrylamide gel electrophoresis (SDS-PAGE) under reducing and non-reducing conditions. The protein was stored at -80°C in TBS-CaCl (0.13 M NaCl, 20 mM Tris, 1 mM CaCl, pH 7.4). Urokinase was separated from the human serum albumin stabilizer by affinity chromatography on a cyanogen bromide activated Sepharose 4B column to which polyclonal rabbit anti-human albumin antibody had been coupled. The urokinase-albumin mixture was repeatedly chromatographed until the urokinase in the fall through fraction was electrophoretically devoid of albumin.

Protein concentrations were determined according to, Bradford, M.M., Anal. Biochem., 72:248-254 (1976), or by determining absorbance at 280 nm. Detergent containing samples were determined by the method of Smith, P.K. et al., Anal. Biochem., 150:76-85 (1985).

SDS-PAGE was performed according to Laemmli, Nature, 227:680-685 (1970) and proteins were visualized with Coomassie Brilliant Blue G-250 according to Neuhoff, V. et al., Electrophoresis, 9:255-262 (1988) or with silver staining. Merril, C.R. et al., Science, 211:1437 (1981).

### C. Preparation of 7E3-Fab'

The antiplatelet monoclonal antibody 7E3-Fab' was prepared by reducing 7E3-(Fab')₂ (10 mg, 2 mg/ml 0.1 M sodium phosphate, pH 6.8) at room temperature for 18 hours in 1 mM 2-mercaptoethylamine, 1 mM ethylene diamine tetraacetic acid and 10 mM sodium arsenite, followed by the addition of solid Ellman's reagent to a concentration of 5 mM. After 30 minutes at room temperature, excess reagent was removed by gel filtration on a Sephadex® G-25 column (30 x 2) cm, equilibrated with 0.1 M sodium phosphate (pH 6.8). In this protected form 7E3-Fab' has remained structurally and functionally intact at 4°C under sterile conditions for over 3 months. The thiol form of 7E3-Fab' was easily regenerated by treatment with 10 mM 2-mercaptoethylamine for 30 minutes at room temperature, followed by gel filtration.

### D. Preparation of rtPA-7E3-Fab' Conjugate and Urokinase-7E3-Fab' Conjugate

Urokinase (4 ml) in NaPi buffer (3.0 mg/ml) in 0.14 M NaCl, 3.7 mM sodium phosphate, 1 mM KCl, pH 7.4) was mixed with SPDP (20 mM in absolute ethanol, 5-fold molar excess added dropwise to the stirred solution) and the reaction was allowed to proceed for 30 minutes at room temperature. The reaction mixture was then dialysed overnight against several changes of 0.1 M sodium phosphate (pH 6.8). Analysis for 2-pyridyldisulfide content (Stuchbury, Biochem. J., 151:417 (1975)) showed typically about 0.5 to 2.0 residues per urokinase molecule. The substitution level was kept intentionally low in order to interfere as little as possible with the specific properties of urokinase and to avoid the formation of higher molecular weight aggregates.

Recombinant tPA (5.7 mg, 1.0 mg/ml) was equilibrated with NaPi buffer by gel filtration on Sephadex® G-25 immediately before modification. SPDP was added as described above. After 30 minutes, the reaction mixture was immediately dialysed against NaPi buffer containing 1.0 M arginine and 0.1% Tween® 80 for 2 changes over 12 hours at 4°C. Runge, M.S. et al., Proc. Natl. Acad. Sci. USA, 84:7659-7662 (1987).

The thiol form of 7E3-Fab' (5.0 ml at 1.0 mg/ml in sodium phosphate, pH 6.8) was mixed with either SPDP modified urokinase or modified rtPA and allowed to react for 20 hours at room temperature. The reaction was stopped by the addition of 100-fold molar excess (compared with protein concentrations) of iodoacetamide in 0.1 M sodium phosphate (pH 8.0).

E. Purification of Conjugates

The desired conjugates were purified from the reaction mixtures in two sequential affinity chromatography steps. First, selection for plasminogen activator binding to benzamidine-Sepharose® retained conjugate and unconjugated plasminogen activator, but not uncoupled 7E3-Fab' (which was also recovered). The eluate (elution buffer: 0.1 M sodium acetate, 0.4 M sodium chloride, pH 4.0) was neutralized with 3 M Tris-HCl pH 8.0 and passed through a column of Sepharose® conjugated to a goat anti-mouse-Fab-antibody. This column retained only the desired conjugate which was eluted with 0.2 M glycine, pH 2.8, subsequently neutralized with 3 M Tris-HCl sodium chloride, 0.01% Tween® 80 and 0.01% sodium azide). Conjugates could be stored in this buffer under sterile conditions at 4°C for at least 4 weeks.

The nature of the disulfide linked urokinase-7E3-Fab' and rtPA-7E3-Fab' conjugates is defined in part by their purification. Because of the two step affinity purification procedure, conjugates must contain both the ability to bind to benzamidine (i.e., serine protease activity) and the ability to bind to a goat anti-mouse-fab antibody (i.e., contain a fab-portion). When electrophoresed under reducing conditions, the disulfide bond is broken, and one can visualize the individual components (single-chain rtPA at 66KD or urokinase heavy and light chain at 34 KD and 21 KD, respectively, Fab'-heavy chain at 28 KD and light chain at 25 KD). Under non-reducing conditions molecules of an apparent molecular weight of approximately 110 KD and 100 KD were visualized.

F. Quantitation of Urokinase and rtPA Activities

Urokinase and rtPA activities were determined as described in Runge et al., Biochem, 27:1153-1157 (1988).

Briefly, standardized samples of UK in Plough units and milligrams per milliliter and of single-chain recombinant tPA in International units and milligrams per milliliter were analyzed in a chromogenic substrate assay (S-2288 in 0.15 M Tris and 0.15 M NaCl, pH 8.4, with a substrate concentration of $1 \times 10^{-3}$ mol/L and an enzyme concentration of $8 \times 10^{-9}$ mol/L). For UK, 1 Plough unit was assumed to equal $1.8 \times 10^{-4}$ nmol; for tPA, 1 International unit was assumed to equal $6.3 \times 10^{-5}$ nmol. The correlation between the change in absorbance per minute reported for the S-2288 assay and that for our samples was excellent, such that 100 units of UK ($8 \times 10^{-5}$ mmol) or 100 units of single-chain tPA ($6.3 \times 10^{-6}$ mmol) gave a change in absorbance at 405 nm of approximately 0.060/min. On the basis of these results, whenever a determination was made on the activity (in appropriate units as above) or molar amount (in millimoles) of active enzyme in an unknown sample of UK or tPA, the sample was diluted until an absorbance change of 0.060/min. at 405 nm occurred in an S-2288 assay, as described above. The linear range of enzyme concentration to absorbance change was from $4 \times 10^{-10}$ mol/L to $3.2 \times 10^{-8}$ mol/L in our hands. If concentrations above this range were used in assays for fibrinolysis, appropriate dilutions were made from stock solutions containing higher concentrations (aliquots of which had been tested at a 1:10 dilution in the S-2288 assay).

In order to get an even more realistic comparison of the plasminogen activation properties of either urokinase or rtPA and their respective conjugates, enzyme activity was monitored with the plasmin sensitive chromogenic substrate S-2251. 96-well cell culture plates (Linbro) were coated with TBS-CaCl containing 5% (w/v) bovine serum albumin (BSA) to inhibit unspecific absorption. The plates were then extensively rinsed with water and dried. 50 Il plasminogen activator solution, containing different amounts of enzyme, were added to the wells. Then, a substrate solution, consisting of 1 part 3.5 mM S-2251 in H2O and 3 parts (v/v) plasminogen (0.75 CU/ml) in S-2251 assay buffer (50 mM Tris, 110 mM sodium chloride) was mixed and 100 Il of this solution were added to each well. The plasmin dependent conversion of substrate S-2251 was read at 405 nm in a conventional Elisa-reader. Medac urokinase was used as a standard.

Example II: Plasminogen Activation in the Presence of whole GP IIb/IIIa Complex

Ninety-six well, U-shaped microtiter plates (Becton Dickinsen) were coated overnight at 4°C with 100 Il GP IIb/IIIa solution (50 Ig/ml). The plates were extensively rinsed with water and 200 Il TBS-CaCl containing 1% BSA were added to block unspecific binding. After 6 hours at room temperature the plates were rinsed again. 100 ll of TBS-CaCl containing different amounts of either rtPA or urokinase or the respective conjugates were then added to each well for 4 hours at room temperature. The plates were rinsed again and 100 ul of substrate solution, composed as described above, were added to each well. The increase in absorbance at 405 nm indicating the presence of plasmin was measured after 90 minutes and 120 minutes.

Urokinase, rtPA and their conjugates were compared in the presence of GP IIb/IIIa as follows: the plasminogen activation potential of each activator was first assessed in the absence of GP IIb/IIIa as described previously. Samples were diluted so that the activator concentration ranges from 0.1 units to 10 units /100 ll. Table 1 shows, that after 90 minutes, considerably more plasmin has been generated by the conjugates than by their respective parent molecule. At 120 minutes UK-7E3-Fab' is almost 20 times as potent as UK and rtPA-7E3-Fab' is between 20 and 25 times as potent as uncoupled rtPA. Urokinase is less potent than rtPA by a factor of less than 4, UK-7E3-Fab' is not quite 5 times as potent as uncoupled rtPA.

Example III: Plasminogen Activation in the Presence of Whole Platelets

Platelet concentrates were stored at -80°C. After thawing they were centrifuged at 1800 g for 15 minutes at 4°C. The pellet, containing the platelets, was washed three times in TBS-EDTA (0.13 M NaCl, 20 mM Tris, 1 mM EDTA, pH 7.4). The platelets in the final pellet were resuspended in TBS-CaCl to a concentration of approximately 100,000 platelets per ll. 100 ll of platelet suspension were added to each well of a 96-well microtiter plate. The plates were centrifuged at 2000 g for 10 minutes. DeMarco, L. et al., Acta Haemat., 75:203-206 (1986). After washing the plates with water 2 times, 200 ll TBS-CaCl containing 5% BSA (w/v) were added to each well. After 6 hours at room temperature the plates were washed again with water. At this point, the coated plates could be stored for up to 1 week at 4°C. Then, plasminogen activator in TBS-CaCl containing 2.5% BSA were added to each well and incubated for 3 hours at room temperature. After extensive rinsing with water, substrate solution was added and the increase in absorbance at 405 nm indicating the presence of plasmin was measured after 90 and 120 minutes.

Table 2 illustrates that plasminogen conversion to plasmin was markedly enhanced for both conjugates in comparison to the native plasminogen activators. The enhancement factors appear to be somewhat lower than in the presence of purified IIb/IIIa which can probably be explained by the fact that less IIb/IIIa molecules are available for binding in this assay. Moreover, in this assay, rtPA appears to be a more efficient plasminogen activator than UK7E3-Fab'

Example IV: Clot Lysis Assay in Buffer and Human Plasma

The method of Lijnen et al. was used with some modifications. Lijnen et al., Thromb. Haemostas, 52: 208-310 (1984). Five units of fresh frozen plasma obtained from the local blood bank were pooled, aliquoted and refrozen. Immediately before each experiment the activities of the various plasminogen activators were calibrated as described above. The plasminogen activation potential in the absence of the antibody target molecule was determined and related to that of a standard concentration of urokinase. Appropriate dilutions were made so that the plasminogen activation was identical for each sample. Platelet concentrates were washed as described above at room temperature. The final pellet was resuspended in the same volume of plasma. Platelet rich clots were made by adding to the platelet suspension, in this sequence, 125-I-labelled human fibrinogen (500,000 cpm/ml of suspension), 0.5 M CaCl (final concentration in plasma, 0.05 M) and thrombin (8 NIH units/ml of plasma). Immediately after the addition of thrombin, the solution was drawn into a Silastic tubing (inner diameter of 4 mm) and allowed to clot for 30 minutes at 37°C. The tubing was then cut into sections of 2.5 cm, yielding clots of approximately 0.2 ml. The clots were removed from the tubing and each was placed in a plastic vial and washed with 3 ml of 0.15 M NaCl. They were subsequently counted and suspended in either 2 ml of TBS containing plasminogen (0.1 mg/ml) or 2 ml of fresh frozen plasma (of the same pool). Experiments were started by the addition of plasminogen activators (or TBS as control) in 200 ll of TBS. At various intervals aliquots of the plasminogen solution (or of fresh frozen plasma) were removed from each tube for counting. Lysis was determined from the calculated total amount of radioactivity released from the clot and radioactivity initially incorporated into the clot.

Urokinase and its conjugate were compared in an assay for human clot lysis. First, lysis was performed on a platelet rich clot in a plasminogen containing buffer. Table 3 shows that the conjugate proved to effect consistently more lysis at equal concentrations than plasminogen activator. The activity ratio was determined to be approximately 2

Table 4 shows that when the modified Lijnen et al. method was repeated using human plasma environment rather than TBS-plasminogen buffer, UK-7E3-Fab' enhanced thrombolysis by an even larger factor. Urokinase was almost inactive at the concentrations used, but UK-7E3-Fab' proved to be an efficient lytic agent.

The preceding examples demonstrate:

1. that antibody 7E3 can be coupled to either urokinase (UK) or tissue plasminogen activator (tPA) to form an immunoconjugate;

2. that the antibody or coupled fab'-fragment moiety retains it capability to bind to platelets and the plasminogen activator moiety retains its ability to activate plasminogen;

3. that the activation of plasminogen can be concentrated to the vicinity of the target protein IIb/IIIa; and

4. that the activation of plasminogen can be concentrated to the vicinity of whole platelets.

5. that activator concentration is possible in human plasma (with all inhibitors present) and that enhanced thrombolysis of a platelet rich thrombus can be effected.

## TABLE 1

## Assay with Conjugates in GP IIa/IIIa Coated Wells

## Increase in absorbance after 90 minutes

| Units/well | | Urokinase | Urokinase-7E39(Fab') | tPA | tPA-7E3 (Fab') |
|---|---|---|---|---|---|
| 10 | | 0.117 | 1.772 | 0.250 | 1.523 |
| | | 0.074 | 1.831 | 0.243 | 1.663 |
| | | 0.061 | 1.908 | 0.181 | 1.621 |
| | | 0.053 | 1.722 | 0.237 | 1.623 |
| | mean | 0.076 | 1.808 | 0.227 | 1.607 |
| | SD | 0.024 | 0.069 | 0.027 | 0.051 |
| 2.5 | | 0.008 | 0.528 | 0.060 | 1.144 |
| | | 0.030 | 0.629 | 0.047 | 1.155 |
| | | 0.019 | 0.591 | 0.059 | 1.146 |
| | | 0.013 | 0.611 | 0.062 | 1.152 |
| | mean | 0.017 | 0.589 | 0.057 | 1.149 |
| | SD | 0.008 | 0.088 | 0.005 | 0.004 |
| 0.5 | | 0.004 | 0.062 | -0.003 | 0.331 |
| | | -0.024 | 0.069 | -0.016 | 0.389 |
| | | -0.013 | 0.028 | -0.024 | 0.376 |
| | | 0.017 | 0.049 | -0.011 | 0.385 |
| | mean | -0.004 | 0.052 | -0.013 | 0.036 |
| | SD | 0.015 | 0.015 | 0.007 | 0.023 |
| 0.1 | | 0.005 | 0.046 | 0.016 | 0.036 |
| | | 0.003 | 0.021 | -0.016 | 0.065 |
| | | 0.025 | -0.002 | -0.030 | 0.032 |
| | | -0.004 | 0.021 | -0.024 | 0.051 |
| | mean | 0.007 | 0.021 | -0.013 | 0.046 |
| | SD | 0.010 | 0.016 | 0.017 | 0.013 |

Table 1 (cont.)

## Increase in absorbance after 120 minutes

| Units/well | | Urokinase | Urokinase-7E39(Fab') | tPA | tPA-7E3 (Fab') |
|---|---|---|---|---|---|
| 10 | | 0.184 | 2.338 | 0.436 | 2.191 |
| | | 0.111 | 2.259 | 0.455 | 2.156 |
| | | 0.106 | 2.278 | 0.317 | 2.173 |
| | | 0.096 | 2.111 | 0.479 | 2.048 |
| | mean | 0.124 | 2.246 | 0.421 | 2.142 |
| | SD | 0.034 | 0.083 | 0.062 | 0.055 |
| 2.5 | | 0.009 | 0.919 | 0.090 | 1.774 |
| | | 0.039 | 1.016 | 0.077 | 1.748 |
| | | 0.029 | 1.011 | 0.091 | 1.744 |
| | | 0.022 | 1.012 | 0.097 | 1.720 |
| | mean | 0.024 | 0.989 | 0.088 | 1.746 |
| | SD | 0.010 | 0.040 | 0.007 | 0.019 |
| 0.5 | | 0.000 | 0.085 | -0.010 | 0.571 |
| | | -0.036 | 0.091 | -0.023 | 0.643 |
| | | -0.012 | 0.044 | -0.041 | 0.610 |
| | | 0.013 | 0.062 | -0.030 | 0.640 |
| | mean | -0.008 | 0.070 | -0.026 | 0.616 |
| | SD | 0.018 | 0.018 | 0.011 | 0.029 |
| 0.1 | | 0.005 | 0.045 | 0.007 | 0.054 |
| | | -0.006 | 0.011 | -0.034 | 0.099 |
| | | 0.021 | -0.011 | -0.047 | 0.056 |
| | | -0.001 | 0.027 | -0.041 | 0.063 |
| | mean | 0.004 | 0.018 | -0.028 | 0.068 |
| | SD | 0.010 | 0.020 | 0.021 | 0.018 |

9

## TABLE 2

## Assays with Conjugates in Whole Platelet Coated Wells

Increase in absorbance after 120 minutes

| Units/ well | | Urokinase | Urokinase- 7E39 (Fab') | tPA | tPA-7E3 (Fab') |
|---|---|---|---|---|---|
| 10 | | 0.046 | 0.229 | 1.091 | 1.689 |
| | | 0.040 | 0.173 | 1.185 | 1.550 |
| | mean | 0.042 | 0.201 | 1.138 | 1.619 |
| | SD | 0.003 | 0.028 | 0.047 | 0.069 |
| 2.5 | | −0.023 | 0.009 | 0.064 | 0.883 |
| | | 0.006 | 0.004 | 0.085 | 0.835 |
| | mean | −0.008 | 0.006 | 0.074 | 0.859 |
| | SD | 0.014 | 0.002 | 0.010 | 0.024 |
| 0.5 | | −0.003 | 0.004 | −0.003 | 0.247 |
| | | −0.024 | 0.009 | −0.005 | 0.323 |
| | mean | −0.013 | 0.006 | −0.004 | 0.285 |
| | SD | 0.010 | 0.002 | 0.001 | 0.038 |
| 0.1 | | −0.016 | −0.016 | −0.006 | 0.098 |
| | | −0.018 | 0.003 | 0.003 | 0.177 |
| | mean | −0.017 | −0.006 | −0.001 | 0.137 |
| | SD | 0.001 | 0.009 | 0.004 | 0.039 |

Table 2 (Cont.)

Increase in absorbance after 210 minutes

| Units/ well | | Urokinase | Urokinase- 7E39(Fab') | tPA | tPA-7E3 (Fab') |
|---|---|---|---|---|---|
| 10 | | 0.204 | 0.830 | 2.069 | 2.385 |
| | | 0.205 | 0.788 | 2.175 | 2.066 |
| | mean | 0.204 | 0.809 | 2.122 | 2.225 |
| | SD | 0.000 | 0.021 | 0.053 | 0.159 |
| 2.5 | | 0.013 | 0.143 | 0.328 | 2.036 |
| | | 0.026 | 0.112 | 0.341 | 1.979 |
| | mean | 0.019 | 0.127 | 0.334 | 2.007 |
| | SD | 0.006 | 0.015 | 0.006 | 0.028 |
| 0.5 | | 0.001 | 0.022 | 0.023 | 0.890 |
| | | -0.020 | 0.032 | 0.025 | 1.166 |
| | mean | -0.009 | 0.027 | 0.024 | 1.028 |
| | SD | 0.010 | 0.005 | 0.001 | 0.138 |
| 0.1 | | -0.016 | -0.009 | 0.034 | 0.457 |
| | | -0.008 | 0.006 | 0.029 | 0.787 |
| | mean | -0.012 | -0.001 | 0.031 | 0.622 |
| | SD | 0.004 | 0.007 | 0.002 | 0.165 |

## TABLE 3

### Platelet-Rich-Clot Lysis Assay in TBS-Plasminogen

| Lysis Units/clot | | Percent Lysis at 19h | |
|---|---|---|---|
| | | Urokinase | Urokinase-7E3(Fab') |
| 50 | | 42.1 | 64.1 |
| | | 36.8 | 43.3 |
| | | 42.3 | 34.6 |
| | mean | 40.4 | 47.3 |
| | SD | 2.5 | 12.4 |
| 20 | | 28.0 | 37.6 |
| | | 26.6 | 30.9 |
| | | 26.3 | 34.2 |
| | mean | 27.0 | 34.2 |
| | SD | 0.7 | 2.7 |
| 10 | | 25.9 | 45.9 |
| | | 26.2 | 32.7 |
| | | 23.6 | 28.1 |
| | mean | 25.3 | 35.6 |
| | SD | 1.1 | 7.5 |
| 5 | | 24.3 | 26.4 |
| | | 11.9 | 28.2 |
| | | 20.1 | 20.2 |
| | mean | 18.8 | 25.0 |
| | SD | 5.1 | 3.3 |
| 2 | | 9.9 | 23.2 |
| | | 6.6 | 14.3 |
| | | 6.6 | 14.8 |
| | mean | 7.7 | 17.4 |
| | SD | 1.5 | 4.0 |

**Table 3 (Cont.)**

| Lysis Units/clot | | Percent Lysis at 19h | |
|---|---|---|---|
| | | Urokinase | Urokinase-7E3(Fab') |
| 1 | | 1.0 | 12.9 |
| | | 1.7 | 13.1 |
| | | 2.0' | 12.4 |
| | mean | 1.9 | 12.8 |
| | SD | 0.1 | 0.2 |

Table 3 (Cont.)

| Lysis Units/clot | | Percent Lysis at 32h | |
|---|---|---|---|
| | | Urokinase | Urokinase-7E3(Fab') |
| 50 | | 67.4 | 80.1 |
| | | 65.8 | 64.8 |
| | | 66.0 | 55.3 |
| | mean | 66.4 | 66.8 |
| | SD | 0.7 | 10.2 |
| 20 | | 51.5 | 54.9 |
| | | 51.4 | 50.4 |
| | | 49.9 | 57.8 |
| | mean | 50.9 | 54.4 |
| | SD | 0.7 | 3.0 |
| 10 | | 47.4 | 64.1 |
| | | 57.6 | 53.0 |
| | | 48.0 | 52.7 |
| | mean | 51.1 | 56.6 |
| | SD | 4.6 | 5.3 |
| 5 | | 44.4 | 53.8 |
| | | 28.5 | 54.5 |
| | | 39.0 | 36.4 |
| | mean | 37.3 | 48.2 |
| | SD | 6.5 | 8.3 |
| 2 | | 35.8 | 55.2 |
| | | 17.5 | 36.1 |
| | | 16.9 | 33.7 |
| | mean | 23.4 | 41.7 |
| | SD | 8.7 | 9.6 |
| 1 | | 11.4 | 28.4 |
| | | 3.2 | 31.3 |
| | | 4.2 | 34.5 |

Table 3 (Cont.)

| Lysis Units/clot | | Percent Lysis at 32h | |
|---|---|---|---|
| | | Urokinase | Urokinase-7E3(Fab') |
| | mean | 5.5 | 21.4 |
| | SD | 3.4 | 2.4 |

## TABLE 4

### Platelet-Rich-Clot Lysis Assay in Human Plasma

| Lysis Units/clot | | Percent Lysis at 6h | |
| --- | --- | --- | --- |
| | | Urokinase | Urokinase-7E3(Fab') |
| 250 | | 1.6 | 17.4 |
| | | 1.1 | 14.3 |
| | | 1.3 | 15.2 |
| | mean | 1.3 | 15.6 |
| | SD | 0.2 | 1.3 |
| 200 | | 0.8 | 9.4 |
| | | 0.7 | |
| | | 1.0 | |
| | mean | 0.8 | |
| | SD | 0.1 | |
| 150 | | 0.7 | 10.8 |
| | | 0.8 | 10.4 |
| | | 0.7 | 12.4 |
| | mean | 0.8 | 11.2 |
| | SD | 0.0 | 0.8 |
| 100 | | 0.6 | 4.9 |
| | | 0.7 | 3.1 |
| | | 0.9 | 8.2 |
| | mean | 0.7 | 5.4 |
| | SD | 0.1 | 2.1 |
| 50 | | 0.6 | 3.9 |
| | | 0.6 | 3.8 |
| | | 0.7 | 4.2 |
| | mean | 0.6 | 4.0 |
| | SD | 0.0 | 0.1 |

Table 4 (cont.)

## Platelet-Rich-Clot Lysis Assay in Human Plasma

| Lysis Units/clot | | Percent Lysis at 24h | |
|---|---|---|---|
| | | Urokinase | Urokinase-7E3(Fab') |
| 250 | | 1.7 | 31.0 |
| | | 1.5 | 27.0 |
| | | 1.8 | 29.0 |
| | mean | 1.7 | 29.1 |
| | SD | 0.1 | 1.5 |
| 200 | | 1.2 | 17.4 |
| | | 1.2 | 19.7 |
| | | 1.2 | 19.3 |
| | mean | 1.2 | 18.8 |
| | SD | 0.0 | 0.8 |
| 150 | | 1.1 | 18.9 |
| | | 0.9 | 18.0 |
| | | 1.3 | 20.6 |
| | mean | 1.1 | 19.2 |
| | SD | 0.1 | 1.0 |
| 100 | | 1.0 | 6.6 |
| | | 1.0 | 5.4 |
| | | 1.2 | 14.7 |
| | mean | 1.0 | 8.9 |
| | SD | 0.0 | 4.1 |
| 50 | | 0.9 | 6.1 |
| | | 0.8 | 6.0 |
| | | 1.0 | 7.2 |
| | mean | 0.9 | 6.4 |
| | SD | 0.0 | 0.5 |

Equivalents

Those skilled in the art will recognize or be able to ascertain, using no more than routine experimentation, many equivalents to the specific embodiments of the invention described herein. Such equivalents are intended to be encompassed by the following claims.

**Claims**

**Claims for the following Contracting States : AT, BE, CH, DE, DK, FR, GB, IT, LI, LU, NL, SE**

1. An immunoconjugate comprising a platelet-specific antibody or fragment thereof (e.g. a monoclonal antibody or fragment thereof) and a thrombolytic agent.

2. An immunoconjugate of claim 1, wherein the monoclonal antibody or fragment thereof specifically binds the glycoprotein IIb/IIIa receptor complex of platelets, and for example wherein the monoclonal antibody is 7E3.

EP 0 465 556 B1

3. An immunoconjugate of claim 1, wherein the platelet-specific monoclonal antibody is of human origin.

4. An immunoconjugate of claim 1, wherein the antibody fragment is selected from Fab, Fab' and F(ab')$_2$.

5. An immunoconjugate of claim 1, wherein the thrombolytic agent is selected from streptokinase, urokinase, tissue plasminogen activator, single chain streptokinase, acyl plasminogen streptokinase activator complex, and recombinant variants of the thrombolytic agents or fragments thereof.

6. An immunoconjugate of claim 1 produced by recombinant techniques.

7. An immunoconjugate comprising a Fab' fragment of monoclonal antibody 7E3 coupled to a thrombolytic agent, by for example recombinant techniques.

8. An immunoconjugate comprising a Fab' antibody fragment derived from a human monoclonal antibody specific for GPIIb/IIIc receptor coupled to a thrombolytic agent.

9. An immunoconjugate of claim 7 or claim 8, wherein the thrombolytic agent is (a) tissue-type plasminogen activator; or (b) urokinase.

10. An immunoconjugate according to claim 1 for use in administration to a patient having a thrombus or at risk of thrombus formation.

11. An immunoconjugate according to claim 10, wherein (a) the antibody or antibody fragment is specific for the GPIIb/IIIa receptor; or (b) the antibody fragment is a Fab, Fab' or F(ab')$_2$ fragment; or (c) thrombolytic agent is selected from tissue plasminogen activator, streptokinase, single chain streptokinase, acyl-plasminogen-streptokinase activator complex, urokinase and recombinant variants of the thrombolytic agents or fragments thereof.

**Claims for the following Contracting State : ES**

1. A process of making an immunological agent comprising coupling a platelet-specific antibody or fragment thereof (e.g. a monoclonal antibody or fragment thereof) with a thrombolytic agent.

2. A process of claim 1, wherein the chosen monoclonal antibody or fragment thereof specifically binds the glycoprotein IIb/IIIa receptor complex of platelets, and for example wherein the monoclonal antibody is 7E3.

3. A process of claim 1, wherein the platelet-specific monoclonal antibody is of human origin.

4. A process of claim 1, wherein the antibody fragment is selected from Fab, Fab' and F(ab')$_2$.

5. A process of claim 1, wherein the thrombolytic agent is selected from streptokinase, urokinase, tissue plasminogen activator, single chain streptokinase, acyl plasminogen streptokinase activator complex, and recombinant variants of the thrombolytic agents or fragments thereof.

6. A process of claim 1 which involves recombinant techniques.

7. A process of making an immunological agent, comprising coupling a Fab' fragment of monoclonal antibody 7E3 to a thrombolytic agent, by for example recombinant techniques.

8. A process of making an immunological agent, comprising coupling a Fab' antibody fragment derived from a human monoclonal antibody specific for GPIIb/IIIc receptor to a thrombolytic agent.

9. A process of claim 7 or claim 8, wherein the thrombolytic agent is (a) tissue-type plasminogen activator; or (b) urokinase.

10. Use of the product of the process of claim 1 for the manufacture of a medicament for administration to a patient having a thrombus or at risk of thrombus formation.

16

EP 0 465 556 B1

11. Use according to claim 10, wherein (a) the antibody or antibody fragment is specific for the GPIIb/IIIa receptor; or (b) the antibody fragment is a Fab, Fab' or F(ab')$_2$ fragment; or (c) thrombolytic agent is selected from tissue plasminogen activator, streptokinase, single chain streptokinase, acyl-plasminogen-streptokinase activator complex, urokinase and recombinant variants of the thrombolytic agents or fragments thereof.

**Patentansprüche**
**Patentansprüche für folgende Vertragsstaaten : AB, BE, CH, DE, DK, FR, GB, IT, LI, LU, NL, SE**

1. Ein Immunokonjugat, das einen blutplättchenspezifischen Antikörper oder dessen Fragment (z. B. einen monoklonalen Antikörper oder dessen Fragment) und ein thrombolytisches Agens umfaßt.

2. Ein Immunokonjugat des Anspruchs 1, worin der monoklonale Antikörper oder dessen Fragment den Glykoprotein IIb/IIIa Rezeptorkomplex der Blutplättchen spezifisch bindet, und worin beispielsweise der monoklonale Antikörper 7E3 ist.

3. Ein Immunokonjugat des Anspruchs 1, worin der blutplättchenspezifische Antikörper humanen Ursprungs ist.

4. Ein Immunokonjugat des Anspruchs 1, worin das Antikörperfragment Fab, Fab' oder F(ab')$_2$ ist.

5. Ein Immunokonjugat des Anspruchs 1, worin das thrombolytische Agens Streptokinase, Urokinase, Gewebe-Plasminogen-Aktivator, Einstrang-Streptokinase, acylierter Plasminogen-Streptokinase-Aktivator-Komplex oder rekombinante Varianten der thrombolytischen Agentien oder deren Fragmente ist.

6. Ein Immunokonjugat des Anspruchs 1, das mittels Rekombinationsverfahren hergestellt ist.

7. Ein Immunokonjugat, das ein Fab' Fragment des monoklonalen Antikörpers 7E3 umfaßt, das an ein thrombolytisches Agens mittels beispielsweise Rekombinationsverfahren gekoppelt ist.

8. Ein Immunokonjugat, das ein Fab' Antikörperfragment umfaßt, das von einem für den GPIIb/IIIc Rezeptor spezifischen humanen monoklonalen Antikörper stammt und an ein thrombolytisches Agens gekoppelt ist.

9. Ein Immunokonjugat des Anspruchs 7 oder Anspruchs 8, worin das thrombolytische Agens (a) ein Gewebetyp-Plasminogen-Aktivator; oder (b) Urokinase ist.

10. Ein Immunokonjugat nach Anspruch 1 für die Verwendung zur Verabreichung an einen Patienten mit einem Thrombus oder mit dem Risiko einer Thrombusbildung.

11. Ein Immunokonjugat nach Anspruch 10, worin (a) der Antikörper oder das Antikörperfragment spezifisch für den GPIIb/IIIa Rezeptor ist; oder (b) das Antikörperfragment ein Fab, Fab' oder F(ab')$_2$ Fragment ist; oder (c) das thrombolytische Agens ein Gewebe-Plasminogen-Aktivator, Streptokinase, Einstrang-Streptokinase, acylierter Plasminogen-Streptokinase-Aktivator-Komplex, Urokinase oder rekombinante Varianten der thrombolytischen Agentien oder deren Fragmente ist.

**Patentansprüche für folgenden Vertragsstaat : ES**

1. Ein Verfahren zur Herstellung eines immunologischen Agens, das die Kopplung eines blutplättchenspezifischen Antikörpers oder dessen Fragments (z. B. eines monoklonalen Antikörpers oder dessen Fragments) mit einem thrombolytischen Agens umfaßt.

2. Ein Verfahren des Anspruchs 1, worin der ausgewählte monoklonale Antikörper oder dessen Fragment den Glykoprotein IIb/IIIa Rezeptorkomplex der Blutplättchen spezifisch bindet, und worin beispielsweise der monoklonale Antikörper 7E3 ist.

3. Ein Verfahren des Anspruchs 1, worin der blutplättchenspezifische Antikörper humanen Ursprungs ist.

17

**4.** Ein Verfahren des Anspruchs 1, worin das Antikörperfragment Fab, Fab' oder F(ab')$_2$ ist.

**5.** Ein Verfahren des Anspruchs 1, worin das thrombolytische Agens Streptokinase, Urokinase, Gewebe-Plasminogen-Aktivator, Einstrang-Streptokinase, acylierter Plasminogen-Streptokinase-Aktivator-Komplex oder rekombinante Varianten der thrombolytischen Agentien oder deren Fragmente ist.

**6.** Ein Verfahren des Anspruchs 1, das Rekombinationsverfahren umfaßt.

**7.** Ein Verfahren zur Herstellung eines immunologischen Agens, das die Kopplung eines Fab' Fragments des monoklonalen Antikörpers 7E3 an ein thrombolytisches Agens beispielsweise mittels Rekombinationsverfahren umfaßt.

**8.** Ein Verfahren zur Herstellung eines immunologischen Agens, das die Kopplung eines Fab' Antikörperfragments, das von einem für den GPIIb/IIIc Rezeptor spezifischen humanen monoklonalen Antikörper stammt, an ein thrombolytisches Agens umfaßt.

**9.** Ein Verfahren des Anspruchs 7 oder Anspruchs 8, worin das thrombolytische Agens (a) ein Gewebetyp-Plasminogen-Aktivator; oder (b) Urokinase ist.

**10.** Die Verwendung des Produkts des Verfahrens des Anspruchs 1 zur Herstellung eines Medikaments zur Verabreichung an einen Patienten mit einem Thrombus oder mit dem Risiko einer Thrombusbildung.

**11.** Die Verwendung nach Anspruch 10, worin (a) der Antikörper oder das Antikörperfragment spezifisch für den GPIIb/IIIa Rezeptor ist; oder (b) das Antikörperfragment ein Fab, Fab' oder F(ab')$_2$ Fragment ist; oder (c) das thrombolytische Agens ein Gewebe-Plasminogen-Aktivator, Streptokinase, Einstrang-Streptokinase, acylierter Plasminogen-Streptokinase-Activator-Komplex, Urokinase oder rekombinante Varianten der thrombolytischen Agentien oder deren Fragmente ist.

**Revendications**

**Revendications pour les Etats contractants suivants : AT, BE, CH, DE, DK, FR, GB, IT, LI, LU, NL, SE**

**1.** Immunoconjugué comprenant un anticorps ou un fragment d'anticorps spécifique des plaquettes (par exemple un anticorps ou fragment d'anticorps monoclonal) et un agent thrombolytique.

**2.** Immunoconjugué selon la revendication 1, dans lequel l'anticorps ou le fragment d'anticorps monoclonal se lie de manière spécifique au complexe récepteur glycoprotéine IIb/IIIa des plaquettes, et par exemple dans lequel l'anticorps monoclonal est 7E3.

**3.** Immunoconjugué selon la revendication 1, dans lequel l'anticorps monoclonal spécifique des plaquettes est d'origine humaine.

**4.** Immunoconjugué selon la revendication 1, dans lequel le fragment d'anticorps est choisi parmi Fab, Fab' et F(ab')$_2$.

**5.** Immunoconjugué selon la revendication 1, dans lequel l'agent thrombolytique est choisi parmi la streptokinase, l'urokinase, l'activateur tissulaire de plasminogène, la streptokinase monocaténaire, le complexe activateur acyl plasminogène-streptokinase, et des variants recombinants des agents ou fragments d'agents thrombolytiques.

**6.** Immunoconjugué selon la revendication 1, produit par des techniques recombinantes.

**7.** Immunoconjugué selon la revendication 1, comprenant un fragment Fab' d'anticorps monoclonal 7E3 couplé à un agent thrombolytique, par exemple par des techniques recombinantes.

**8.** Immunoconjugué comprenant un fragment d'anticorps Fab' dérivé d'un anticorps monoclonal humain spécifique pour le récepteur GPIIb/IIIa couplé à un agent thrombolytique.

9. Immunoconjugué selon la revendication 7 ou la revendication 8, dans lequel l'agent thrombolytique est (a) l'activateur de plasminogène de type tissulaire; ou (b) l'urokinase.

10. Immunoconjugué selon la revendication 1, destiné à être administré à un patient présentant un thrombus ou un risque de formation de thrombus.

11. Immunoconjugué selon la revendication 10, dans lequel (a) l'anticorps ou le fragment d'anticorps est spécifique pour le récepteur GPIIb/IIIa; ou (b) le fragment d'anticorps est un fragment Fab, Fab' ou F-(ab')$_2$; ou (c) l'agent thrombolytique est choisi parmi l'activateur tissulaire de plasminogène, la streptokinase, la streptokinase monocaténaire, le complexe activateur acyl plasminogène-streptokinase, l'urokinase et les variants recombinants des agents ou fragments d'agents thrombolytiques.

**Revendications pour l'Etat contractant suivant : ES**

1. Procédé de préparation d'un agent immunologique comprenant le couplage d'un anticorps ou fragment d'anticorps spécifique des plaquettes (par exemple un anticorps ou fragment d'anticorps monoclonal) avec un agent trombolytique.

2. Procédé selon la revendication 1, dans lequel l'anticorps ou le fragment d'anticorps monoclonal choisi se lie de façon spécifique au complexe récepteur glycoprotéine IIb/IIIa des plaquettes, et par exemple dans lequel l'anticorps monoclonal est 7E3.

3. Procédé selon la revendication 1, dans lequel l'anticorps monoclonal spécifique des plaquettes est d'origine humaine.

4. Procédé selon la revendication 1, dans lequel le fragment d'anticorps est choisi parmi Fab, Fab' et F-(ab')$_2$.

5. Procédé selon la revendication 1, dans lequel l'agent thrombolytique est choisi parmi la streptokinase, l'urokinase, l'activateur tissulaire de plasminogène, la streptokinase monocaténaire, le complexe activateur acyl plasminogène-streptokinase, et des variants recombinants des agents ou fragments d'agents thrombolytiques.

6. Procédé selon la revendication 1, mettant en oeuvre des techniques recombinantes.

7. Procédé de préparation d'un agent immunologique, comprenant le couplage d'un fragment Fab' d'anticorps monoclonal 7E3 à un agent thrombolytique, par exemple par des techniques recombinantes.

8. Procédé de préparation d'un agent immunologique, comprenant le couplage d'un fragment d'anticorps Fab' dérivé d'un anticorps monoclonal humain spécifique pour le récepteur GPIIb/IIIa, à un agent thrombolytique.

9. Procédé selon la revendication 7 ou la revendication 8, dans lequel l'agent thrombolytique est (a) l'activateur de plasminogène de type tissulaire; ou (b) l'urokinase.

10. Utilisation du produit du procédé de la revendication 1, pour la préparation d'un médicament destiné à être administré à un patient présentant un thrombus ou un risque de formation de thrombus.

11. Utilisation selon la revendication 10, dans laquelle (a) l'anticorps ou le fragment d'anticorps est spécifique pour le récepteur GPIIb/IIIa; ou (b) le fragment d'anticorps est un fragment Fab, Fab' ou F-(ab')$_2$; ou (c) l'agent thrombolytique est choisi parmi l'activateur tissulaire de plasminogène, la streptokinase, la streptokinase monocaténaire, le complexe activateur acyl plasminogène-streptokinase, l'urokinase et des variants recombinants des agents ou fragments d'agents thrombolytiques.